# EUROPEAN PATENT SPECIFICATION

(11) **EP 2 758 404 B1**
(45) Date of publication and mention of the grant of the patent: **09.01.2019**
(21) Application number: 12756714.7
(22) Date of filing: 07.09.2012
(51) Int. Cl.: C07D 493/04, A61Q 19/10, A61Q 19/00, A61K 8/49

(54) **USE OF ISOSORBIDE ALKYL MONOESTERS AS FOAM IMPROVERS IN AQUEOUS COSMETIC PREPARATIONS**
VERWENDUNG VON ISOSORBID-ALKYL-MONOESTERN ALS SCHAUMVERSTÄRKER IN WÄSSRIGEN KOSMETIKPRÄPARATEN
UTILISATION DE MONOESTERS D'ISOSORBIDE ALKYLIQUES EN TANT QUE RENFORÇATEURS DE MOUSSE DANS DES PRÉPARATIONS COSMÉTIQUES AQUEUSES

(30) Priority: 19.09.2011 EP 11181837
(43) Date of publication of application: 30.07.2014
(73) Proprietor: BASF SE, 67056 Ludwigshafen am Rhein (DE)
(72) Inventor: STOER, Claudia, 40597 Düsseldorf (DE); DIERKER, Markus, 40593 Düsseldorf (DE); NIEENDICK, Claus, 47807 Krefeld (DE); SEIPEL, Werner, 40723 Hilden (DE); BEHLER, Ansgar, 46240 Bottrop (DE); PRINZ, Daniela, 41542 Dormagen (DE); BREFFA, Catherine, 68159 Mannheim (DE); WEISSENEGGER, Markus, 40627 Düsseldorf (DE); RATHS, Hans-Christian, 40789 Monheim (DE)
(74) Representative: BASF IP Association
(86) International application number: PCT/EP2012/067481
(87) International publication number: WO 2013/041388

(56) References cited:
- EP-A1- 2 174 641
- WO-A1-01/83488
- WO-A1-2008/095571
- WO-A1-2010/040464
- WO-A2-2011/059866
- JP-A- 8 173 787
- DATABASE CA [Online] CHEMICAL ABSTRACTS SERVICE, COLUMBUS, OHIO, US; 1989, VILL, V. ET AL: "Helical twisting power of carbohydrate derivatives", XP002661914, retrieved from STN Database accession no. 1989:163966 & VILL, V. ET AL: "Helical twisting power of carbohydrate derivatives", ZEITSCHRIFT FUER NATURFORSCHUNG, A: PHYSICAL SCIENCES, vol. 43, no. 12, 1988, pages 1119-1125, ISSN: 0932-0784
- DATABASE CA [Online] CHEMICAL ABSTRACTS SERVICE, COLUMBUS, OHIO, US; 1955, HAYASHI, IZUMI ET AL: "Preparation of sorbide esters and their properties as plasticizers", XP002661915, retrieved from STN Database accession no. 1955:46014 & HAYASHI, IZUMI ET AL: "Preparation of sorbide esters and their properties as plasticizers", NIPPON KAGAKU KAISHI (1921-47) , IND. CHEM. SECT., vol. 56, 1953, pages 623-625, ISSN: 0369-4208
- DATABASE CA [Online] CHEMICAL ABSTRACTS SERVICE, COLUMBUS, OHIO, US; 1997, CHALECKI, ZBIGNIEW ET AL: "Lipozyme-mediated regioselective esterification of isosorbide under solvent-free conditions", XP002661916, retrieved from STN Database accession no. 1997:707515 & CHALECKI, ZBIGNIEW ET AL: "Lipozyme-mediated regioselective esterification of isosorbide under solvent-free conditions", SYNTHETIC COMMUNICATIONS, vol. 27, no. 22, 1997, pages 3847-3852, ISSN: 0039-7911
- PAUL BECHER: "NON-IONIC SURFACE-ACTIVE COMPOUNDS. III. EFFECT OF STRUCTURE ON MICELLE FORMATION IN BENZENE SOLUTION", JOURNAL OF PHYSICAL CHEMISTRY, vol. 64, 1960, pages 1221-1223, XP002661917, ISSN: 0022-3654
- DATABASE CA [Online] CHEMICAL ABSTRACTS SERVICE, COLUMBUS, OHIO, US; 2008, YANG, LI-QUN ET AL: "Synthesis and properties of liquid crystalline monomers, and polymers based on an isosorbide derivative", XP002661918, retrieved from STN Database accession no. 2008:170957 & YANG, LI-QUN ET AL: "Synthesis and properties of liquid crystalline monomers, and polymers based on an isosorbide derivative", GAOFENZI CAILIAO KEXUE YU GONGCHENG, vol. 23, no. 6, 2007, pages 43-45, ISSN: 1000-7555
- DATABASE CA [Online] CHEMICAL ABSTRACTS SERVICE, COLUMBUS, OHIO, US; 1987, SAHEKI, YUKITAMI ET AL: "Synthesis of 2-alkyloyl-1,4,3,6-dianhydrosorbitol-5-sul fates and evaluation of the surface active properties", XP002661919, retrieved from STN Database accession no. 1987:158306 & SAHEKI, YUKITAMI ET AL: "Synthesis of 2-alkyloyl-1,4,3,6-dianhydrosorbitol-5-sul fates and evaluation of the surface active properties", JAOCS, J. AM. OIL CHEM. SOC., vol. 63, no. 7, 1986, pages 927-930,

## Description

The present invention relates to the use of certain derivatives of isosorbide for producing cosmetic compositions.

In the field of cosmetic preparations for skin and haircare, a large number of requirements are imposed by the consumer: apart from the cleaning and care effects, which determine the intended use, value is placed on such differing parameters as best possible dermatological compatibility, good refatting properties, elegant appearance, optimal sensory impression and storage stability.

Preparations which are used for the cleaning and care of human skin and hair generally comprise, alongside a series of surface-active substances, especially oil bodies and water. The oil bodies/emollients used are, for example, hydrocarbons, ester oils and vegetable and animal oils/fats/waxes. In order to meet the high requirements of the market with regard to sensory properties and optimal dermatological compatibility, new oil bodies and emulsifier mixtures are being continually developed and tested. For the production of cosmetic preparations, a large number of natural and synthetic oils, for example almond oil or avocado oil, ester oils, ethers, alkyl carbonates, hydrocarbons, and also silicone oils are used. It is an essential task of the oil components, as well as the care effect, which is directly connected to skin greasing, to impart to the consumer a non-sticky, long-lasting feel of skin smoothness and suppleness which develops as quickly as possible.

As well as oil bodies, further constituents are also used in cosmetic compositions which, for example, influence the foaming behavior and/or the rheology, which serve as emulsifiers for the purpose of stably formulating aqueous and nonaqueous phases alongside one another, or which are able to impart further functionalities, e.g. a pearlescent effect.

WO 2011/059866, EP 2174641 A1, WO 01/83488 describe the use of isosorbide diesters in the cosmetic field. WO 2008/095571 discloses mixtures of isosorbide diesters with carboxylic acids for technical applications such as a plasticizer. Database Chemical Abstract XP 002661914 proposes isosorbide diesters as a doping material for liquid crystal phase. Database Chemical Abstract XP-002661915 describes the preparation of isosorbide diesters as plasticizers.

JP 8173787 deals with isosorbide diethers. WO 2010/040464 describes isosorbide derivatives with alkylene oxide groups for cosmetic applications.

Chemical Abstract 197:707515, describes monoesters of isosorbide wherein R^{II} is a linear alkyl group having 7,9,11,13 and 15 carbon atoms. This abstract reports on lipozyme-mediated regioselective esterification of isosorbide under solvent-free conditions. No application of the monoester claimed is described.

Paul Becher mentions in Journal of Physical Chemistry, Vol. 64, pages 1221-1223 isosorbide monostearate in table I and is silent on any application of this stearate. This paper is concerned with studies on micelle formation in the presence of this isosorbide monostearate.

Chemical Abstract 2008:170957 shows an isosorbide monoester wherein R^{II} is a linear alkyl radical having 10 carbon atoms. This abstract only reports on the synthesis and properties of liquid crystal monomers and polymers based on isosorbide derivatives.

Chemical Abstract 1987:158306, shows isosorbide monoesters wherein R^{II} is a linear alkyl group having 7, 9, 11 and 13 carbon atoms. It is said that the 5-sulfates thereof have surface-active properties.

There is therefore a constant need to provide new ingredients which are suitable for improving the foaming behaviour in cosmetic compositions. It has now been found that derivatives of isosorbide can be used advantageously in cosmetic compositions.

Isosorbide (or 1, 4'; 3,6-dianhydrosorbitol) is the anhydride of sorbitol. It can be obtained, for example, by heating sorbitol in the presence of concentrated sulfuric acid or hydrochloric acid. By means of methods known per se to the person skilled in the art, it is possible to obtain various derivatives of isosorbide, for example ethers, esters or salts.

The present invention relates to the use of isosorbide derivatives according to the general formula (I) 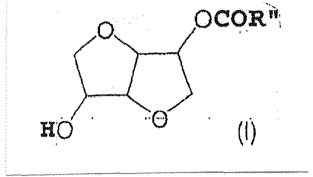
where R" is a linear or branched, saturated or unsaturated
alkyl radical having 5 to 23 carbon atoms,
as a foam improver for producing aqueous cosmetic compositions.

The general formula (I) also includes all stereoisomers of isosorbide, and any desired mixtures thereof. The general formula (I) otherwise includes all combinations of the radicals R and R' among one another.

**Cosmetic compositions** are to be understood here as meaning all compositions known to the person skilled in the art which are exclusively or primarily intended to be used externally on the human body or in its oral cavity for cleaning, care, protection, maintaining a good condition, perfuming, changing the appearance or for the purposes of influencing body odor.

The cosmetic compositions according to the invention can be in particular formulations for bodycare, e.g. a body milk, creams, lotions, sprayable emulsions, products for eliminating body odor etc. The hydrocarbons can also be used in surfactant-containing formulations such as e.g. foam and shower baths, hair shampoos and care rinses. Depending on the intended application, the cosmetic formulations comprise a series of further auxiliaries and additives, such as, for example, surfactants, further oil bodies, emulsifiers, pearlescent waxes, consistency regulators, thickeners, superfatting agents, stabilizers, polymers, fats, waxes, lecithins, phospholipids, biogenic active ingredients, UV light protection factors, antioxidants, deodorants, antiperspirants, antidandruff agents, film formers, swelling agents, insect repellents, self-tanning agents, tyrosine inhibitors (depigmentation agents), hydrotropes, solubilizers, preservatives, perfume oils, dyes etc., which are listed below by way of example.

**Surfactants** Surface-active substances which may be present are anionic, nonionic, cationic and/or amphoteric or zwitterionic surfactants. In surfactant-containing cosmetic preparations, such as, for example, shower gels, foam baths, shampoos etc., preferably at least one anionic surfactant is present. The fraction of surfactants here is usually about 1 to 30, preferably 5 to 25 and in particular 10 to 20% by weight.

Typical examples of **anionic surfactants** are soaps, alkylbenzenesulfonates, alkanesulfonates, olefinsulfonates, alkyl ether sulfonates, glycerol ether sulfonates, α-methyl ester sulfonates, sulfo fatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, such as, for example, acyl lactylates, acyl tartrates, acyl glutamates and acyl aspartates, alkyl oligoglycoside sulfates, protein fatty acid condensates (in particular wheat-based vegetable products) and alkyl (ether) phosphates. If the anionic surfactants contain polyglycol ether chains, these can have a conventional homolog distribution, but preferably have a narrowed homolog distribution. Typical examples of **nonionic surfactants** are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partially oxidized alk(en)yl oligoglycosides and glucuronic acid derivatives, fatty acid N-alkylglucamides, protein hydrolyzates (in particular wheat-based vegetable products), polyol fatty acid esters, sugar esters, sorbitan esters, polysorbates and amine oxides. If the nonionic surfactants contain polyglycol ether chains, these can have a conventional homolog distribution, but preferably have a narrowed homolog distribution. Typical examples of **cationic surfactants** are quaternary ammonium compounds, such as, for example, dimethyldistearylammonium chloride, and ester quats, in particular quaternized fatty acid trialkanolamine ester salts. Typical examples of **amphoteric or zwitterionic surfactants** are alkylbetaines, alkylamidobetaines, aminopropionates, aminoglycinates, imidazoliniumbetaines and sulfobetaines. Said surfactants are exclusively known compounds. As regards structure and preparation of these substances, reference may be made to relevant review works in this field. Typical examples of particularly suitable mild, i.e. particularly skin-compatible, surfacatants are fatty alcohol polyglycol ether sulfates, monoglyceride sulfates, mono- and/or dialkyl sulfosuccinates, fatty acid isethionates, fatty acid sarcosinates, fatty acid taurides, fatty acid glutamates, α-olefinsulfonates, ether carboxylic acids, alkyl oligoglucosides, fatty acid glucamides, alkylamidobetaines, amphoacetals and/or protein fatty acid condensates, the latter preferably based on wheat proteins.

**Oil bodies** Bodycare compositions, such as creams, lotions and milks, usually comprise a series of further oil bodies and emollients which contribute to further optimizing the sensory properties. The oil bodies are usually present in a total amount of 1-50% by weight, preferably 5-25% by weight and in particular 5-15% by weight. As further oil bodies come, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as e.g. myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also of suitability are esters of linear C₆-C₂₂-fatty acids with branched alcohols, in particular 2-ethylhexanol, esters of C₁₈-C₃₈-alkylhydroxycarboxylic acids with linear or branched C₆-C₂₂-fatty alcohols, in particular dioctyl malate, esters of linear and/or branched fatty acids with polyhydric alcohols (such as e.g. propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as e.g. dicaprylyl carbonate (Cetiol® CC), Guerbet carbonates based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as e.g. dicaprylyl ether (Cetiol® OE), ring-opening products of epoxidized fatty acid esters with polyols.

**Fats and waxes** Fats and waxes are added to the bodycare products as care substances and also in order to increase the consistency of the cosmetics. Typical examples of fats are glycerides, i.e. solid vegetable or animal products, which consist essentially of mixed glycerol esters of higher fatty acids. Fatty acid partial glycerides, i.e. technical-grade mono- and/or diesters of glycerol with fatty acids having 12 to 18 carbon atoms, such as, for example, glycerol mono/dilaurate, -palmitate or -stearate are also suitable for this purpose. Suitable waxes are, inter alia, natural waxes, such as e.g. candelilla wax, carnauba wax, japan wax, esparto grass wax, cork wax, guaruma wax, rice germ oil wax, sugar cane wax, ouricury wax, montan wax, beeswax, shellac wax, spermaceti, lanolin (wool wax), uropygial grease, ceresine, ozokerite (earth wax), petrolatum, paraffin waxes, microwaxes; chemically modified waxes (hard waxes), such as e.g. montan ester waxes, sasol waxes, hydrogenated jojoba waxes, and also synthetic waxes, such as e.g. polyalkylene waxes and polyethylene glycol waxes. Besides the fats, suitable additives are also fat-like substances such as lecithins and phospholipids. Examples of natural lecithins which may be mentioned are the cephalins, which are also referred to as phosphatidic acids and are derivatives of 1,2-diacyl-sn-glycerol-3-phosphoric acids. By contrast, phospholipids are usually understood to mean mono- and preferably diesters of phosphoric acid with glycerol (glycerol phosphates), which are generally classed as fats. In addition, sphingosines and sphingolipids are also suitable.

Suitable **thickeners** are, for example, Aerosil grades (hydrophilic silicas), polysaccharides, in particular xanthan gum, guar guar, agar agar, alginates and tyloses, carboxymethylcellulose and hydroxyethyl- and hydroxypropylcellulose, polyvinyl alcohol, polyvinylpyrrolidone and bentonites such as e.g. Bentone® Gel VS-5PC (Rheox).

**UV light protection factors** are to be understood as meaning, for example, organic substances (light protection filters) that are present in liquid or crystalline form at room temperature and which are able to absorb ultraviolet rays and release the absorbed energy again in the form of longer-wave radiation, e.g. heat. UV-B filters can be oil-soluble or water-soluble. Suitable typical UV-A filters are in particular derivatives of benzoylmethane. The UV-A and UV-B filters can of course also be used in mixtures, e.g. combinations of the derivatives of benzoylmethane, e.g. 4-tert-butyl-4'-methoxydibenzoylmethane (Parsol® 1789) and 2-ethylhexyl 2-cyano-3,3-phenylcinnamate (Octocrylene), and esters of cinnamic acid, preferably 2-ethylhexyl 4-methoxycinnamate and/or propyl 4-methoxycinnamate and/or isoamyl 4-methoxycinnamate. Combinations of this type are often combined with water-soluble filters such as e.g. 2-phenylbenzimidazole-5-sulfonic acid and the alkali metal, alkaline earth metal, ammonium, alkylammonium, alkanolammonium and glucammonium salts thereof.

As well as the specified soluble substances, insoluble light protection pigments, namely finely disperse metal oxides, are also suitable. Examples of suitable metal oxides are, in particular, zinc oxide and titanium dioxide. Besides the two aforementioned groups of primary light protection substances, it is also possible to use secondary light protection agents of the antioxidant type; these interrupt the photochemical reaction chain which is triggered when UV radiation penetrates into the skin.

**Biogenic active ingredients** are to be understood as meaning, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, such as e.g. prune extract, bambara nut extract and vitamin complexes.

**Deodorizing active ingredients** counteract, mask or eliminate body odors. Body odors arise as a result of the action of skin bacteria on apocrine perspiration, during which unpleasant smelling degradation products are formed. Accordingly, suitable deodorizing active ingredients are, inter alia, antimicrobial agents, enzyme inhibitors, odor absorbers or odor maskers.

Suitable **insect repellents** are, for example, N,N-diethyl-m-toluamide, 1,2-pentanediol or 3-(N-n-butyl-N-acetylamino)propionic acid ethyl ester), which is sold under the name Insect Repellent® 3535 by Merck KGaA, and also butylacetylaminopropionate.

A suitable **self-tanning agent** is dihydroxyacetone. Suitable tyrosine inhibitors, which prevent the formation of melanin and are used in depigmentation compositions, are, for example, arbutin, ferulic acid, kojic acid, coumaric acid and ascorbic acid (vitamin C).

Suitable **preservatives** are, for example, phenoxyethanol, formaldehyde solution, parabens, pentanediol or sorbic acid, and also the silver complexes known under the name Surfacine®, and the other substance classes listed in Annex 6, Parts A and B of the Cosmetics Ordinance.

**Perfume oils** which may be mentioned are mixtures of natural and synthetic fragrances. Natural fragrances are extracts from flowers, stems and leaves, fruits, fruit peels, roots, woods, herbs and grasses, needles and branches, resins and balsams. Also of suitability are animal raw materials, such as, for example, civet and castoreum, and also synthetic fragrance compounds of the ester, ether, aldehyde, ketone, alcohol and hydrocarbon types.

Suitable **pearlescent waxes,** in particular for use in surface-active formulations, are for example: alkylene glycol esters, specifically ethylene glycol distearate; fatty acid alkanolamides, specifically coconut fatty acid diethanolamide; partial glycerides, specifically stearic acid monoglyceride; esters of polyhydric, optionally hydroxy-substituted carboxylic acids with fatty alcohols having 6 to 22 carbon atoms, specifically long-chain esters of tartaric acid; fatty substances, such as, for example, fatty alcohols, fatty ketones, fatty aldehydes, fatty ethers and fatty carbonates, which have in total at least 24 carbon atoms, specifically laurone and distearyl ether; fatty acids such as stearic acid, hydroxystearic acid or behenic acid, ring-opening products of olefin epoxides having 12 to 22 carbon atoms with fatty alcohols having 12 to 22 carbon atoms and/or polyols (without the sorbitan derivatives) having 2 to 15 carbon atoms and 2 to 10 hydroxyl groups, and mixtures thereof.

**Superfatting agents** which can be used are substances such as, for example, lanolin and lecithin, and also polyethoxylated or acylated lanolin and lecithin derivatives, polyol fatty acid esters, monoglycerides and fatty acid alkanolamides, the latter serving simultaneously as foam stabilizers.

**Stabilizers** which can be used are metal salts of fatty acids, such as e.g. stearates and ricinoleates of magnesium, aluminum and/or zinc.

To improve the flow behavior, **hydrotropes,** such as, for example, ethanol, isopropyl alcohol, or polyols, can also be used. Polyols which are suitable here preferably have 2 to 15 carbon atoms and at least two hydroxyl groups. The polyols can also contain further functional groups, in particular amino groups, and/or be modified with nitrogen.

Isosorbide esters can be synthesized by esterification processes known per se. By way of example, WO 01/83488 discloses a suitable method. Here, monoesters of isosorbide, or mixtures of monoesters, optionally in the presence of unmodified isosorbide, are possible and can be used within the context of the present teaching.

Mixtures of at least two structurally different isosorbide monoesters of the general formula (I) can of course be used alongside one another.

The structure and in particular the length of the alkyl chain of the isosorbide derivatives plays a particular importance for their suitability for producing cosmetic compositions: thus, in the case of the monoesters, advantageous derivatives are those in which the radical R" is a linear, saturated alkyl radical having 10 to 18, in particular 11 to 17 and preferably 12 to 16, carbon atoms.

As regards the amounts in which the isosorbide derivatives according to the above description are used in cosmetic compositions, this depends on the specific formulation and can vary over a wide range. Typical amounts, however, are 0.5 to 30% by weight, preferably in amounts of from 1 to 15% by weight and in particular in amounts of from 1.5 to 5% by weight, in each case based on the total weight of the cosmetic composition.

In one particular embodiment, the present invention relates to the use of the isosorbide derivatives for producing cosmetic compositions which are free from silicone oils.

The isosorbide derivatives are exceptionally suitable for producing aqueous cosmetic compositions and furthermore to those aqueous compositions which also comprise nonionic emulsifiers (without the isosorbide derivatives), hydrocarbons, astringents, dyes, fragrances, propellants, thickeners, and/or pearlizing agents.

The derivatives used in the invention exhibit particularly positive properties which render them suitable as a foam improver for aqueous cosmetic compositions.

The present teaching also provides cosmetic compositions which comprise at least one water phase and one oil phase, where they comprise at least one isosorbide derivative according to the general formula (I).

### Examples

The investigations, described below, relating to the properties of the isosorbide derivatives were carried out. Wherever ingredients are specified, the INCI nomenclature was used.

To determine the viscosity and assess the pearlescence, **isosorbide esters** with a high mono fraction were incorporated with 1% by weight into the formulation described below shown in Table 1 which comprised a dye for the purposes of better assessing the pearlescence. In this connection, the influence of the chain length was investigated.

**Table 1 - Formulation:**

| **Ingredient** | **Fraction in % by weight** |
|---|---|
| Sodium Laureth Sulfate | 8.6 |
| NaCl | 2.5 |
| Coco-Glucoside | 1.6 |
| Cocamidopropyl Betaine | 1.4 |
| Test substance | 1.0 |
| Sicomet amaranth (1% strength aq.) | 0.1 |
| Euxyl K 400 | 0.1 |
| Water | ad 100 |

The viscosity was determined at 20°C using the Brookfield RVT viscometer (spindle: RV spindle No. 5).

The pearlescence was assessed visually by comparison with a standard pearlizing agent (EGDS = Cutina AGS) and evaluated on a scale from 0 (no pearlescence) to 3 (pearlescence comparable with standard).

The mono content given in Table 2 below was determined by means of GC analysis. Missing fractions up to 100% are composed essentially of the diester and, in small amounts, of isosorbide and/or free fatty acid.

**Table 2**

| C chain length | Mono content/SI% | Viscosity/mPas | Pearlescence |
|---|---|---|---|
| 14 | 87 | 29 500 | 0 |
| 16 | 85 | 17 000 | 2 |
| 18 | 77 | 10 300 | 3 |

| | | | |
|---|---|---|---|
| SI = surface integral | | | |

The measurements reveal a relationship between the viscosity and the chain length. The shorter-chain C14-ester again builds up a significantly higher viscosity than the longer-chain C16- and C18-esters. Pearlescence was observed only in the case of the longer-chain C16- and C16-esters. The C18-ester has a comparable pearlescent effect to the standard.

Saturated and unsaturated, linear and branched isosorbide derivatives with different chain lengths and functional groups (esters, ethers (not inventive)) were evaluated regarding their foam properties using the following formulation.

**Table 3 - Formulation Foam Test**

| **ingredient** | **%** |
|---|---|
| Sodium Laureth Sulfate | 10.8 |
| Cocoamidopropyl Betaine | 1.5 |
| Coco-Glucoside | 1.5 |
| Sodium chloride (viscosity adjustment) | 0.9-2.5 |
| Isosorbide derivative | 1 |
| Sodium benzoate | 0.5 |
| Cationic polymer | 0.2 |
| Citric acid | 0.2 |
| water | Up to 100 |

All derivatives have a mono content higher than 75 %. Typical by-products are the corresponding difunctionalized products, remaining starting material (isosorbide) or fatty alcohols (only for ethers).

### Performance test: foam behavior

The foam behavior of four esters and two ethers (not according to the invention) of isosorbide was measured. All formulations were diluted with hard water (ratio 1:4, 30°C) and stirred in a beaker for 10s at 2000 rpm before the foam high was determined. All results were compared, also to a placebo formulation. All measurements were repeated triply and the results were averaged.

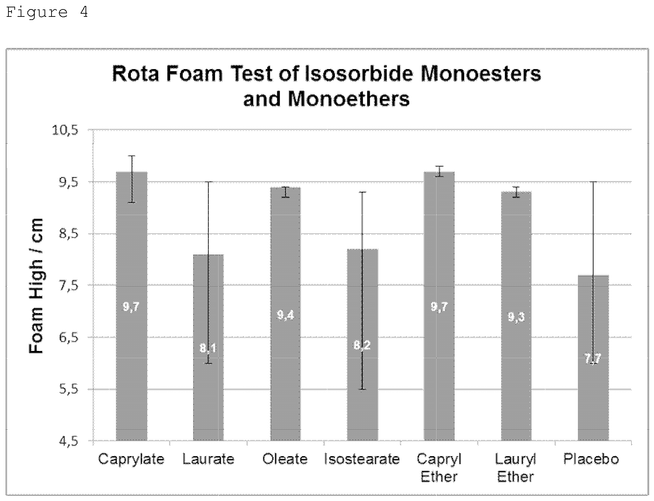

The results have been obtained in a Rota Foam Test and visualized in the diagram of Fig. 4. As can be seen the average amount of foam generated of a formulation containing isosorbide esters or ethers is higher compared to a placebo formulation without additive. The best performance could be observed for shorter chain (capryl ester and ether, lauryl ether) and linear unsaturated derivatives (oleate).

### Carrying out the foam measurement:

The formulations were prepared with 400 g; in each case, 100 g of the formulation were foamed after heating to 30°C. Foaming was effected in an 800 ml beaker for 10 sec at 2000 revolutions using a Meiser disk. The foam height was determined in the beaker. A four-fold determinations were carried out and then averaged.

| **Surfactant** | **Foam height/cm** |
|---|---|
| Sodium Laureth Sulfate | 9.1 |
| Isosorbide based EO-free Sulfate (89% AS) - not inventive -): R = C₁₂H₂₅, R' = SO₃Na | 7.3 |
| Lauryl Glucoside | 6.0 |

| | |
|---|---|
| AS = active substance | |

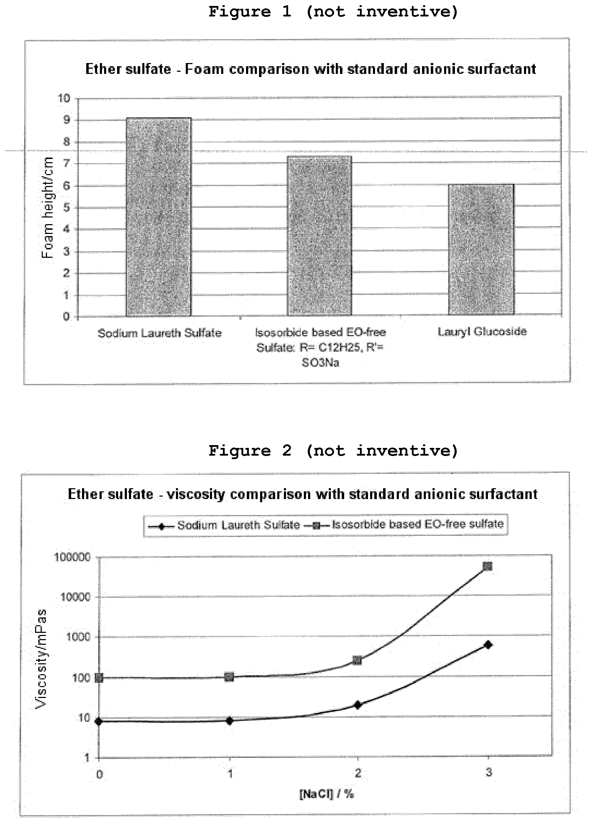

The results can be found in Figure 1: the foaming ability of the EO-free isosorbide ether sulfate is at a high level, even though a lower foam height is reached than with sodium laureth sulfate.

Determination of the thickening with NaCl: To determine the thickenability with sodium chloride, 12% strength aqueous solutions of the test substances with different sodium chloride concentrations were prepared and measured at 22°C using a Brookfield, DV-II + Pro viscometer (spindle LV62 and 60 revolutions for low viscosities, spindle LV64 and 6 revolutions for high viscosities).

| **Anionic surfactant** | **Viscosity/mPas at [NaCl]** | | | |
|---|---|---|---|---|
| | 0 % | 1 % | 2 % | 3 % |
| Sodium Laureth Sulfate | 8 | 8 | 19 | 590 |
| Isosorbide based EO-free Sulfate - not inventive - (89% AS): R = C₁₂H₂₅, R' = SO₃Na | 100 | 100 | 250 | 52 58 9 |

The results are shown in Figure 2. Aqueous solutions which comprise isosorbide ether sulfate can be thickened much more easily with sodium chloride and have a higher starting viscosity. The built-up viscosity is up to two orders of magnitude above that which is achieved with sodium laureth sulfate under identical conditions.

In another experiment, saturated and unsaturated, linear and branched isosorbide derivatives with different chain lengths and functional group (esters, ethers (not inventive)) were evaluated regarding their thickening ability in the presence of sodium chloride using the following formulation.

**Table 4 - Formulation Thickening Test**

| **ingredient** | **%** |
|---|---|
| Sodium Laureth Sulfate | 10.8 |
| Cocoamidopropyl Betaine | 1.5 |
| Coco-Glucoside | 1.5 |
| Sodium chloride | 0.5, 1, 1.5, 2 |
| Isosorbide derivative | 1 |
| Sodium benzoate | 0.5 |
| Cationic polymer | 0.2 |
| Citric acid | 0.2 |
| water | Up to 100 |

For each test substance four formulations with different sodium chloride concentrations were prepared. All derivatives have a mono content higher than 75 %. Typical by-products are the corresponding difunctionalized products, remaining starting material (isosorbide) or fatty alcohols (only for ethers).

The viscosity of all formulations was measured at 20°C using a Brookfield RVF viscometer (spindle number 3-5, 20 rpm).

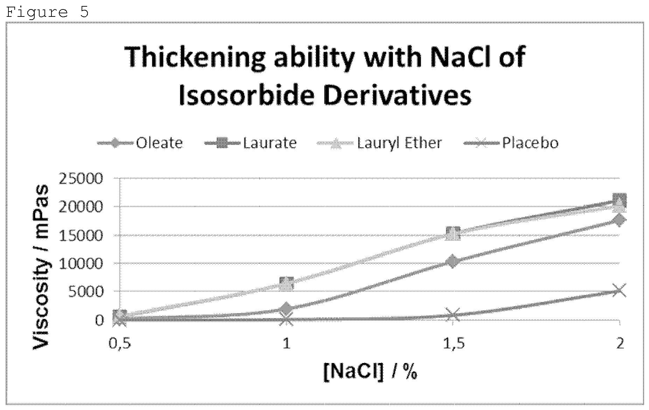

The thickening ability of these isosorbide monoesters and monoethers (not inventive) has been tested. The results are shown in Fig. 5. As can be seen all tested derivatives show a very good thickening performance. Especially the lauryl ester and ether are able to thicken the test formulation already at low sodium chloride concentration.

Various **isosorbide samples** with a different degree of alkylation (ethoxylated or propoxylated; both not inventive)) were assessed in the following formulation according to Table 5 with regard to their foaming ability compared to a placebo formulation (without additive). The results are given in Table 6.

**Table 5 - Formulations:**

| **Ingredient** | **Fraction in % by wt.** |
|---|---|
| Sodium Laureth Sulfate | 10.8 |
| NaCl | 2.4 |
| Test substance | 2 |
| Coco-Glucoside | 1.5 |
| Dist. water | ad 100 |

**Table 6**

| **Test substance** | **Foam height/cm** |
|---|---|
| Isosorbide + 4 EO | 8.2 |
| Isosorbide + 8 EO | 8.4 |
| Isosorbide + 12 EO | 7.7 |
| Isosorbide + 4 EO + 2 PO | 7.8 |
| Isosorbide + 8 EO + 2 PO | 8.6 |
| Isosorbide + 12 EO + 2 PO | 8.6 |
| Placebo | 6.0 |

Carrying out the foam measurement: For the formulations, the individual components were weighed in successively and mixed together. They were then diluted 1:4 with hard water and foamed at 30°C using a Meiser disk in an 800 ml beaker at 2000 revolutions for 10 s. The foam height was measured in the beaker. All of the products exhibited a significant improvement in foam height compared with the formulation without additive.

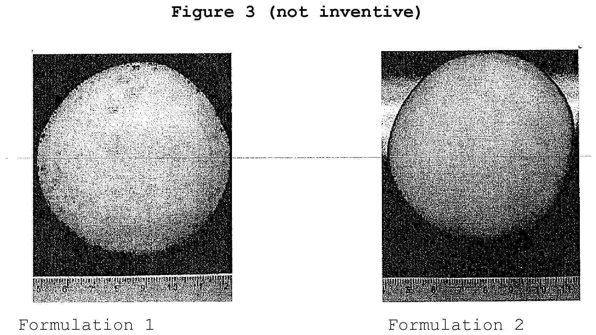

Improvement in foam quality: To assess the foam quality, the following two formulations were prepared, which were then assessed as regards their foam quality in a foam test visually (see photos in Figure 3) by comparison with internal standards:

### Formulation 1 (placebo):

| **Ingredient** | **Fraction in % by wt.** |
|---|---|
| Sodium Laureth Sulfate | 9 |
| Coco-Glucoside | 3 |
| Dist. water | ad 100 |

### Formulation 2 (placebo):

| **Ingredient** | **Fraction in % by wt.** |
|---|---|
| Sodium Laureth Sulfate | 9 |
| Coco-Glucoside | 3 |
| ISB + 4 EO | 2 |
| Dist. water | ad 100% |

Carrying out the foam measurement: For the formulations, the individual components were weighed in successively and mixed together. Then, 17.9 g of the formulation are made up to 100 g with hard water, to give a 2.5% strength solution. The 2.5% strength solution was impacted using a Meiser disk in an 800 ml beaker at 2000 revolutions for 10 s. The foam height was measured in the beaker. The foam was spooned onto a Ceran plate and photographed again. With formulation 1, which comprises no isosorbide ethoxylate, only a qualitatively low quality foam can be produced compared to formulation 2. Accordingly, additives of isosorbide ethoxylate increase the foam quality to a considerable extent.

## Claims

1. Use of isosorbide derivatives according to the general formula (I) 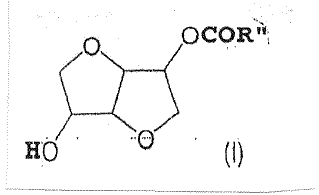 where R" is a linear or branched alkyl radical having 5 to 23 carbon atoms as a foam improver for producing aqueous cosmetic compositions.

2. Use according to Claim 1, **characterized in that** mixtures of at least two structurally different isosorbide derivatives of the general formula (I) are used alongside one another.

3. Use according to Claim 1 and/or claim 2, **characterized in that** R" is a linear, saturated alkyl radical having 10 to 18, in particular 11 to 17 and preferably 12 to 16, carbon atoms.

4. Use according to one or more of Claims 1 to 3, **characterized in that** the isosorbide derivatives are used in amounts of from 0.5 to 30% by weight, preferably in amounts of from 1 to 15% by weight and in particular in amounts of from 1.5 to 5% by weight, in each case based on the total weight of the cosmetic composition.

5. Use according to one or more of Claims 1 to 4, **characterized in that** the isosorbide derivatives are used for producing cosmetic compositions which are free from silicone oils.

6. Use according to one or more of Claims 1 to 5, **characterized in that** the isosorbide derivatives are used for producing aqueous cosmetic compositions which comprise nonionic emulsifiers, hydrocarbons, astringents, dyes, fragrances, propellants, thickeners, and/or pearlizing agents.

## Patentansprüche

1. Verwendung von Isosorbid-Derivaten gemäß der allgemeinen Formel (I) 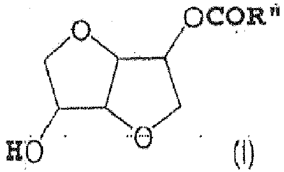 wobei R" für einen linearen oder verzweigten Alkylrest mit 5 bis 23 Kohlenstoffatomen steht, als Schaumverbesserer für die Herstellung von wässrigen kosmetischen Mitteln.

2. Verwendung nach Anspruch 1, **dadurch gekennzeichnet, dass** Mischungen aus mindestens zwei strukturell verschiedenen Isosorbid-Derivaten der allgemeinen Formel (I) nebeneinander verwendet werden.

3. Verwendung nach Anspruch 1 und/oder Anspruch 2, **dadurch gekennzeichnet, dass** R" für einen linearen, gesättigten Alkylrest mit 10 bis 18, insbesondere 11 bis 17 und vorzugsweise 12 bis 16 Kohlenstoffatomen steht.

4. Verwendung nach einem oder mehreren der Ansprüche 1 bis 3, **dadurch gekennzeichnet, dass** die Isosorbid-Derivate in Mengen von 0,5 bis 30 Gew.-%, vorzugsweise in Mengen von 1 bis 15 Gew.-% und insbesondere in Mengen von 1,5 bis 5 Gew.-%, jeweils bezogen auf das Gesamtgewicht des kosmetischen Mittels, verwendet werden.

5. Verwendung nach einem oder mehreren der Ansprüche 1 bis 4, **dadurch gekennzeichnet, dass** die Isosorbid-Derivate zur Herstellung von kosmetischen Mitteln, die frei von Silikonölen sind, verwendet werden.

6. Verwendung nach einem oder mehreren der Ansprüche 1 bis 5, **dadurch gekennzeichnet, dass** die Isosorbid-Derivate zur Herstellung von wässrigen kosmetischen Mitteln, die nichtionische Emulgatoren, Kohlenwasserstoffe, Adstringentien, Farbstoffe, Duftstoffe, Treibmittel, Verdicker und/oder Perlglanzmittel enthalten, verwendet werden.

## Revendications

1. Utilisation de dérivés d'isosorbide répondant à la formule générale (I) 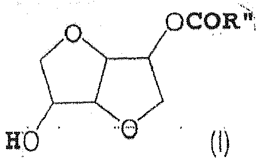 où R" est un radical alkyle linéaire ou ramifié ayant de 5 à 23 atomes de carbone, comme améliorateur de mousse pour la production de compositions cosmétiques aqueuses.

2. Utilisation selon la revendication 1, **caractérisée en ce que** des mélanges d'au moins deux dérivés d'isosorbide différents sur le plan structurel, de formule générale (I), sont utilisés en parallèle.

3. Utilisation selon la revendication 1 et/ou la revendication 2, **caractérisée en ce que** R" est un radical alkyle saturé linéaire ayant de 10 à 18, en particulier de 11 à 17 et préférablement de 12 à 16, atomes de carbone.

4. Utilisation selon l'une ou plusieurs parmi les revendications 1 à 3, **caractérisée en ce que** les dérivés d'isosorbide sont utilisés selon des quantités allant de 0,5 à 30% en poids, préférablement selon des quantités allant de 1 à 15% en poids et en particulier selon des quantités allant de 1,5 à 5% en poids, dans chaque cas sur la base du poids total de la composition cosmétique.

5. Utilisation selon l'une ou plusieurs parmi les revendications 1 à 4, **caractérisée en ce que** les dérivés d'isosorbide sont utilisés pour la production de compositions cosmétiques qui sont dépourvues d'huiles de silicone.

6. Utilisation selon l'une ou plusieurs parmi les revendications 1 à 5, **caractérisée en ce que** les dérivés d'isosorbide sont utilisés pour la production de compositions cosmétiques aqueuses qui comprennent des émulsifiants non ioniques, des hydrocarbures, des astringents, des colorants, des fragrances, des propulseurs, des épaississants, et/ou des agents nacrants.
